(19) Europäisches Patentamt
European Patent Office
Office européen des brevets

(11) Publication number : 0 564 397 A1

(12) # EUROPEAN PATENT APPLICATION

(21) Application number : 93810142.5

(22) Date of filing : 26.02.93

(51) Int. Cl.[5] : **C07D 513/04,** C07D 239/78, A61K 31/505, // (C07D513/04, 277:00, 239:00)

(30) Priority : **03.03.92 US 845123**

(43) Date of publication of application : **06.10.93 Bulletin 93/40**

(84) Designated Contracting States : **AT BE CH DE DK ES FR GB GR IE IT LI LU NL PT SE**

(71) Applicant : **SANDOZ LTD.**
**Lichtstrasse 35**
**CH-4002 Basel (CH)**
(84) **BE CH DK ES FR GB GR IE IT LI LU NL PT SE**

(71) Applicant : **SANDOZ-PATENT-GMBH**
**Humboldtstrasse 3**
**D-79539 Lörrach (DE)**
(84) **DE**

(71) Applicant : **SANDOZ ERFINDUNGEN VERWALTUNGSGESELLSCHAFT M.B.H.**
**Brunner Strasse 59**
**A-1235 Vienna (AT)**
(84) **AT**

(72) Inventor : **Anderson, Paul LeRoy**
**264 Center Grove Road**
**Randolph, NJ 07869 (US)**
Inventor : **Houlihan, William Joseph**
**15 Raynold Road**
**Mountain Lakes, NJ 07046 (US)**
Inventor : **Kapa, Prasad Koteswara**
**4 Faber Road**
**Parsippany, NJ 07054 (US)**
Inventor : **Kucerovy, Andrew**
**Nine Squire Road**
**Hopatcong, NJ 07843 (US)**
Inventor : **Mattner, Paul Gerard**
**504 Jackson Avenue**
**Dunellen, NJ 08812 (US)**

(54) **Antiatherosclerotic substituted phenylquinazoline derivatives.**

(57) The invention relates to substituted phenylquinazoline derivatives. It concerns the compounds of formula I

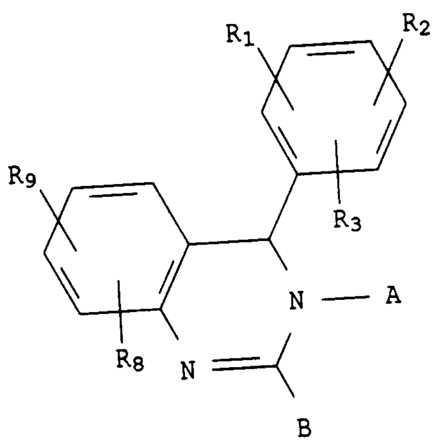

wherein the substituents have various significances.

They may be obtained by a process comprising dehydration, ring closure and/or alkylation, and optional recovery in racemic or optically active, in free base or acid addition salt form.

They possess pharmacological activity, in particular,they raise the blood serum high density lipoprotein level and are thus indicated for use in the treatment of atherosclerosis.

EP 0 564 397 A1

The invention relates to substituted phenylquinazoline derivatives. It concerns the compounds of formula I

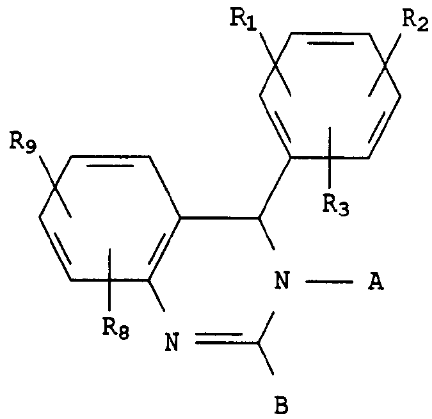

I

wherein

$R_1$ is hydrogen; alkyl of 1 to 3 carbon atoms; alkoxy of 1 to 3 carbon atoms; trifluoromethyl; or fluoro or chloro;

$R_2$ and $R_9$ independently are hydrogen; alkyl of 1 to 3 carbon atoms; alkoxy of 1 or 2 carbon atoms; trifluoromethyl; or fluoro or chloro;

$R_3$ is hydrogen or alkyl of 1 or 2 carbon atoms;

either A and B together are a group of formula $-C(R_4R_5)C(R_6R_7)-S-$ wherein

$R_4$ is hydrogen; alkyl of 1 to 3 carbon atoms optionally mono- or independently di- or independently trisubstituted by halogen of atomic number of from 9 to 35, whereby there is at most 1 bromo; or phenyl optionally mono- or independently disubstituted by alkyl of 1 to 3 carbon atoms, alkoxy of 1 or 2 carbon atoms, or halogen of atomic number of from 9 to 35, whereby there is at most 1 bromo;

$R_5$ is hydroxy and $R_6$ is hydrogen or

$R_5$ and $R_5$ together form a double ($\pi$) bond; and

$R_7$ is hydrogen or alkyl of 1 or 2 carbon atoms;

or A is hydrogen and B is a group of formula $-S-CHR_7'-R_{10}$ wherein

$R_7'$ is hydrogen or alkyl of 1 or 2 carbon atoms and

$R_{10}$ is alkyl of 1 to 3 carbon atoms; alkenyl of 2 or 3 carbon atoms; or $-COOR_{11}$ wherein $R_{11}$ is the residue of an ester group, with the proviso that $R_{10}$ is other than methyl when $R_7'$ is hydrogen;

$R_5$ is hydrogen; alkyl of 1 to 3 carbon atoms; alkoxy of 1 to 3 carbon atoms; trifluoromethyl; or halogen of atomic number of from 9 to 35;

in racemic or optically active, in free base or acid addition salt form,
hereinafter briefly named "the compounds of the invention".

When A and B together are a group of formula $-C(R_4R_5)C(R_5R_7)-S-$it is the carbon atom carrying $R_4$ and $R_5$ which is bound to the nitrogen atom.

A compound of the invention in acid addition salt form preferably is in pharmaceutically acceptable acid addition salt form. By this term is meant an acid addition salt that is physiologically acceptable, i.e. that does not significantly increase the toxicity of the basic compound or otherwise adversely affect its pharmacological activity. Examples are salts with organic acids, e.g. the methanesulfonate, ethanesulfonate, benzenesulfonate, p-toluenesulfonate, acetate, propionate, citrate and tartrate salts, and salts with inorganic acids, e.g. the hydrochloride and sulfate salts. Preferred are the pharmaceutically acceptable monoacid addition salts. Although the compounds of the invention contain two nitrogen atoms, only one is generally protonatable.

The residue of an ester group for significance $R_{11}$ preferably is the residue of a physiologically acceptable ester group. By this term is meant a group which, together with the -COO- radical to which it is attached, forms an ester group which is physiologically acceptable, preferably a physiologically acceptable and hydrolyzable ester group, i.e. a group which, together with the -COO- radical to which it is attached, forms an ester group which is physiologically acceptable and hydrolyzable under physiological conditions to yield a compound wherein $R_{10}$ is replaced by -COOH and an alcohol which itself is physiologically acceptable, i.e. non-toxic at the desired dosage level. The alcohol preferably is free of centers of asymmetry. Examples of such groups

$R_{11}$ are $C_{1-3}$alkyl, n-butyl, i-butyl, t-butyl and benzyl.

Subgroups of compounds of the invention are compounds wherein

- A and B together are a group of formula $-C(R_4R_5)C(R_6R_7)-S-$;
  - in a subgroup thereof (**group Ia**) $R_5$ is hydroxy;
  - in a further subgroup thereof (**group Ib**) $R_5$ and $R_6$ together form a double ($\pi$) bond;
- A is hydrogen and B is a group $-S-CHR_7'-R_{10}$;
  - in a subgroup thereof (**group IIa**) $R_{10}$ is $-COOR_{11}$;
  - in a further subgroup thereof (**group IIb**) $R_{10}$ is other than $-COOR_{11}$.

Each compound of group Ib wherein no substituent contains a center of asymmetry, each compound of group IIa wherein $R_7'$ is hydrogen and the group $-S-CHR_7'-R_{10}$ does not contain a center of asymmetry, and each compound of group IIb wherein $R_7'$ is hydrogen or $R_7'$ and $R_{10}$ are identical, altogether has a single center of asymmetry (the ring carbon atom to which the $R_1$-bearing phenyl group is attached), and, therefore, there are two stereoisomers of each such compound. The two stereoisomers are designated as the 5R and 5S enantiomers in the case of the compounds of group Ib and the 4R and 4S enantiomers in the case of the aforementioned compounds of groups IIa and IIb.

Each compound of group Ia wherein $R_7$ is hydrogen and no further substituent contains a center of asymmetry, altogether has two centers of asymmetry (the aforementioned ring carbon atom and the ring carbon atom to which $R_4$ is attached), and three, four or five centers of asymmetry when $R_4$ contains one or two centers of asymmetry and/or $R_7$ is $C_{1-2}$alkyl (the aforementioned two ring carbon atoms and the ring carbon atom to which $R_7$ is attached and/or any asymmetric carbon atoms in $R_4$).

Each compound of group IIa wherein $R_7'$ is hydrogen and $R_{11}$ contains a single center of asymmetry or wherein $R_7'$ is $C_{1-2}$alkyl and $R_{11}$ is free of centers of asymmetry, has two centers of asymmetry, and each compound of group IIb wherein $R_7'$ is $C_{1-2}$alkyl and $R_{10}$ has a significance that differs from that of $R_7'$ likewise has two centers of asymmetry (the carbon atom to which the $R_1$-bearing phenyl group is attached and the carbon atom to which $R_7'$ is attached or the center of asymmetry in $R_{11}$). Each compound of group IIa wherein $R_7'$ is $C_{1-2}$alkyl and $R_{11}$ contains at least one center of asymmetry, or wherein $R_7'$ is hydrogen and $R_{11}$ contains at least two centers of asymmetry, has three or more centers of asymmetry.

All stereoisomers of each compound of the invention are within the scope of this invention. Preferred are those compounds containing one center of asymmetry. Generally, insofar as the compounds of group Ib are concerned, the 5R enantiomers and 5R,5S racemates are preferred over the corresponding 5S enantiomers, with the 5R enantiomers being preferred over the corresponding racemates. Also generally, insofar as the compounds of groups IIa and IIb having just one center of asymmetry are concerned, the 4R enantiomers and 4R,4S racemates are preferred over the corresponding 4S enantiomers, with the 4R enantiomers being preferred over the corresponding racemates. Insofar as the compounds that contain more than one center of asymmetry are concerned, these preferences represent the preferred configuration at the carbon atom to which the $R_1$-bearing phenyl group is attached.

Generally, the free bases of the compounds of group Ib are preferred over the corresponding acid addition salts, and the acid addition salts of the compounds of groups Ia, IIa and IIb are preferred over the corresponding free bases.

$R_1$ preferably is hydrogen. $R_2$ preferably is hydrogen. $R_3$ preferably is hydrogen. $R_4$ preferably is hydrogen or alkyl of 1 to 3 carbon atoms, it more preferably is methyl or ethyl, especially methyl. $R_5$ and $R_6$ preferably together form a double ($\pi$) bond. $R_7$ preferably is hydrogen. $R_7'$ preferably is hydrogen. $R_5$ preferably is hydrogen or chloro, especially hydrogen. When it is other than hydrogen the substituent preferably is bound to the aromatic ring carbon atom in para position to the nitrogen atom that is part of a double bond in formula I. $R_9$ preferably is hydrogen. $R_{10}$ preferably is alkyl of 2 or 3 carbon atoms, alkenyl of 2 or 3 carbon atoms or $-COOR_{11a}$ wherein $R_{11a}$ is alkyl of 1 to 3 carbon atoms, n-butyl, i-butyl, t-butyl or benzyl; $R_{10}$ is more preferably alkenyl of 2 or 3 carbon atoms or $-COOR_{11a}$, even more preferably vinyl or $-COOR_{11b}$ wherein $R_{11b}$ is alkyl of 1 to 3 carbon atoms; $R_{10}$ is still more preferably $-COOR_{11b}$; it is most preferably methoxycarbonyl or ethoxycarbonyl, especially methoxycarbonyl.

When $R_4$ is optionally mono- or disubstituted phenyl it preferably is unsubstituted phenyl.

A and B preferably are together a group of formula $-C(R_4R_5)C(R_6R_7)-S-$.

Alkyl of 1 to 3, or of 1 or 2 carbon atoms preferably is methyl. Alkoxy of 1 to 3 or of 1 or 2 carbon atoms preferably is methoxy. Halogen of atomic number of from 9 to 35, preferably is chlorine. Alkenyl of 2 or 3 carbon atoms preferably is vinyl.

A further subgroup of compounds of the invention is the compounds of formula Is

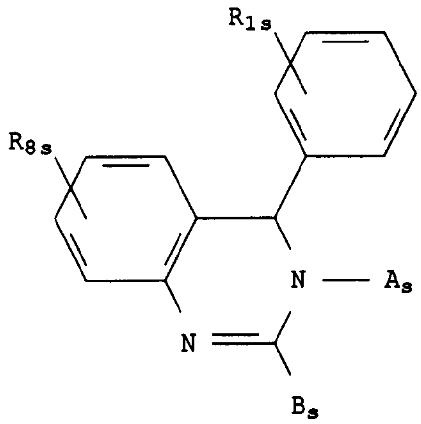

Is

wherein

$R_{1s}$ is hydrogen, fluoro or chloro;

either $A_s$ and $B_s$ together are a group of formula $-C(R_{4s}R_5)CH(R_6)-S-$ wherein

$R_{4s}$ is hydrogen; alkyl of 1 or 2 carbon atoms optionally monosubstituted by bromo; or phenyl; and

$R_6$ and $R_6$ are as defined above;

or $A_s$ is hydrogen and $B_s$ is a group of formula $-S-CH_2-R_{10s}$ wherein

$R_{10s}$ is alkyl of 2 or 3 carbon atoms, alkenyl of 2 or 3 carbon atoms or $-COOR_{11s}$ wherein $R_{11s}$ is alkyl of 1 or 2 carbon atoms;

$R_{6s}$ is hydrogen; alkyl of 1 or 2 carbon atoms; or halogen of atomic number of from 9 to 35;

in racemic or optically active, in free base or acid addition salt form.

When $A_s$ and $B_s$ together are a group of formula $-C(R_{4s}R_5)CH(R_6)-S-$it is the carbon atom carrying $R_{4s}$ and $R_6$ which is bound to the nitrogen atom. $R_{4s}$ preferably is methyl. $R_{10s}$ preferably is ethyl, vinyl or methoxycarbonyl.

In a further subgroup of compounds of the invention $R_7$ and $R_7'$ are other than hydrogen. In a further subgroup $R_{10}$ is other than methyl. In a further subgroup $R_{10}$ is other than alkyl of 1 to 3 carbon atoms. In a further subgroup $R_{10}$ is $-COOR_{11}$. In a further subgroup $R_1$, $R_2$ and $R_3$ are hydrogen. In a further subgroup $R_1$, $R_2$, $R_3$, $R_6$ and $R_9$ are hydrogen.

The compounds of the invention may be prepared by a process which comprises

a) for the preparation of the compounds of formula I wherein $R_5$ and $R_6$ together form a double ($\pi$) bond, dehydrating a corresponding compound of formula I wherein $R_6$ is hydroxy and $R_6$ is hydrogen;

b) for the preparation of the compounds of formula I as defined above under formula I, submitting to appropriate ring closure a compound of formula II

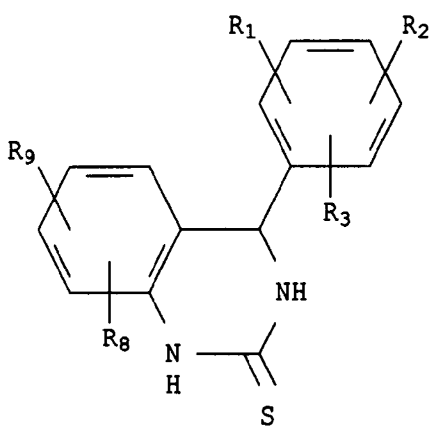

II

wherein the substituents are as defined above under formula I; or

c) for the preparation of the compounds of formula I wherein A is hydrogen and B is a group of formula $-S-CHR_7'-R_{10}$, appropriately alkylating a corresponding compound of formula II, e.g. with a corresponding compound of formula III

$$X'-CHR_7'-R_{10} \qquad III$$

wherein X' is a reactive group and $R_7$' and $R_{10}$ are as defined above;
and recovering the resultant compounds of formula I in racemic or optically active, in free base or acid addition salt form.

The above process for the preparation of the compounds of the invention may be effected in conventional manner.

Process **variant a) (dehydration)** is effected in an inert anhydrous organic solvent such as 2-propanol or toluene. The temperature preferably is from about 60° to about 120°C, preferably from 80° to 83°C or 100° to 112°C. Particularly when $R_4$ is hydrogen it may be indicated to convert to the free base prior to dehydration.

Process **variant b) (ring closure)** is e.g. effected with a compound of formula

$R_4$-CO-CH$R_7$-X, preferably $R_{4x}$-CO-CH$R_7$-X, or of formula $(RO)_2$CH-CH$R_7$-X wherein

| | |
|---|---|
| $R_4$ and $R_7$ | are as defined above, |
| $R_{4x}$ | with the exception of hydrogen has the significance indicated above for $R_4$, |
| X | is a reactive group, preferably halogen of atomic number of from 17 to 53, especially bromo, and |
| R | is methyl or ethyl, preferably ethyl. |

It thus includes a preliminary alkylation step. For the preparation of the compounds wherein $R_5$ is hydroxy and $R_6$ is hydrogen, the reaction, when a compound of formula $R_4$-CO-CH$R_7$-X is used, preferably is effected at a temperature of from about -20° to about +50°C, preferably starting at about -20° to -10°C, allowing to warm to 20°-25°C, and completing the reaction at that temperature. The reaction preferably is effected in an inert organic solvent such an ether solvent, especially tetrahydrofuran or a mixture of diethyl ether and tetrahydrofuran. Preferably an inert atmosphere is used. When a compound of formula $(RO)_2$CH-CH$R_7$-X is used, preferably the temperature is from about 50° to about 80°C. The solvent preferably is a mixture of an ether solvent and water. For the preparation of the compounds wherein $R_5$ and $R_5$ together form a double ($\pi$) bond, preferably a compound of formula $R_{4x}$-CO-CH$R_7$-X is used. When X is chloro, it is preferred to use a catalytic amount of sodium iodide or, preferably, bromide. The temperature preferably is from about 70° to about 80°C. The inert organic solvent preferably is a $C_{2-4}$ alkanol, especially 2-propanol. An inert atmosphere is preferred.

Process **variant c) (alkylation)** preferably is effected with a group $R_{10}$ containing no substituent that would interfere with the reaction. When $R_{10}$ is a group -COO$R_{11}$ which might so interfere, then it may be indicated to effect the alkylation with a reagent containing a group -COO$R_{11}$ which does not interfere, and to transesterify to another group -COO$R_{11}$ after alkylation has taken place. X' preferably is halogen of atomic number of from 17 to 53, preferably bromo. The alkylation reaction preferably is effected at a temperature of from about 20° to about 75°C. Preferably an anhydrous inert organic solvent is used, especially an ether solvent such a diethyl ether or especially tetrahydrofuran. Preferably the reaction is effected in an inert atmosphere. **Transesterification,** if indicated, may e.g. be effected in an inert aqueous organic solvent, e.g. a mixture of water and a lower alkanol such as methanol or, especially, ethanol; it is normally effected in two steps, saponification e.g. with a strong inorganic base such as sodium or potassium hydroxide, at a temperature of from about O° to about 75°C, preferably 20°-50°C, followed by the addition of sufficient acid, e.g. 2 N hydrochloric acid, to lower the pH to 2-4.5, at a temperature of 20°-25°C, isolation of the free acid, and then esterification at a temperature of from about 20° to about 40°C, preferably 20°-25°C, with alcohol $R_{11}$OH and a catalytic amount of a strong acid, e.g. p-toluenesulfonic acid or hydrate thereof, in an inert anhydrous organic solvent, e.g. an ether solvent such as tetrahydrofuran or alcohol $R_{11}$OH, if a liquid.

A compound of the invention may be recovered from the reaction mixture and purified in conventional manner.

A compound in free base form may be converted in conventional manner into a salt form and vice-versa. For the compounds of the invention wherein $R_5$ and $R_5$ together from a double ($\pi$) bond the free base may be recovered from a salt form e.g. by neutralization with an inorganic base, preferably sodium bicarbonate, sodium carbonate or sodium hydroxide. The temperature preferably is from about 15° to about 50°C, preferably from 20° to 45°C. It is effected e.g. in water or a mixture of water and an organic solvent such as ethyl acetate. For the compounds wherein $R_5$ is hydroxy and $R_6$ is hydrogen neutralization is e.g. effected in an organic base, e.g. a tri-($C_{1-3}$alkyl)amine, preferably triethylamine. The temperature is from about 0° to about 25°C, preferably from 20° to 25°. An anhydrous organic solvent preferably is used, preferably a hydrocarbon such as toluene. For the neutralization of compounds wherein B is a group -S-CH$R_7$'-$R_{10}$, e.g. either set of conditions above may be used.

An acid addition salt form of a compound of the invention may be recovered from the free base form e.g. by treating a solution or suspension of the free base in a solvent such as a lower alkanol, e.g. methanol or ethanol, or a mixture thereof with water, at, for example, from about 5° to about 50°C, preferably 10° to 40°C, with, for example, 0.98-6 equivalents of a pharmaceutically acceptable acid for, for example, 2 to 150 minutes, optionally extracting the reaction mixture with an organic solvent that is immiscible therewith, and concentrating or evaporating the reaction mixture at reduced pressure to obtain the acid addition salt. Preferably, however, irrespective of whether the acid is monovalent, divalent, trivalent, etc., 0.98 to 2, preferably 0.99 to 1.02, moles of the acid per mole of the compound of the invention in free base form is employed to obtain a monoacid addition salt.

The compounds of the invention, particularly those wherein R5 is other than hydroxy, may be obtained in optically pure form by utilizing optically pure starting materials. Alternatively, a racemic form of a compound of the invention may be converted into an optically active form in conventional manner. Thus, each compound having one center of asymmetry may be resolved into two optically active isomers by conventional techniques. For example, it may be reacted with an optically pure carboxylic acid having at least one center of asymmetry to form a mixture of diastereoisomeric salts that may be separated e.g. by fractional crystallization or column chromatography. After separation, the optically pure salt may be reconverted to the corresponding compound in free base form by conventional means with retention of optical purity. Any compound of the invention having two (or more) centers of asymmetry, e.g. the compounds wherein $R_5$ is hydroxy, may be separated into four (or more) stereoisomers by conventional techniques. For example, diastereoisomers may be separated by fractional crystallization, column chromatography, preparative thin layer chormatography and HPLC, and each obtained racemate may be resolved as set forth above.

If the compounds wherein $R_5$ is hydroxy are not easily separable, they may be reacted with a carboxylic acid or, preferably, a reactive carboxylic acid derivative such as a halide or anhydride, optionally containing a center of asymmetry, to form the corresponding esters. The esters may be separated as set forth above and, after separation, reconverted to the compounds of the invention with retention of optical purity.

The compounds having one center of asymmetry are preferably resolved by selectively precipitating one enantiomer from a solution containing the D-(-)- or L-(+)-tartrate salts of a racemic compound having just one center of asymmetry, i.e. from a solution containing substantially equal amounts of the D-(-)-tartrate or L-(+)-tartrate salts of the 5R and 5S enantiomers of such a compound, or from a solution of such salts enriched with one enantiomer. The precipitated salt contains at least 75 %, more preferably at least 98 % and especially at least 99.5 % of a single enantiomer. The precipitated salt is then neutralized to obtain the corresponding free base having the same enantiomeric purity.

This procedure is particularly applicable to the compounds of the invention wherein $R_5$ and $R_6$ together form a double ($\pi$) bond, especially the compound of Example 2. Preferably, D-(-)-tartaric acid is utilized when it is desired to selectively precipitate a salt of the 5R enantiomer of these compounds and thereby obtain the 5R enantiomer, and L-(+)-tartaric acid to obtain the 5S enantiomer, particularly for the compound of Example 2.

Preferably, the precipitated tartrate salt of these compounds, particularly when it is the compound of Example 2, is a monoacid addition salt, i.e. a salt wherein one molecule of tartaric acid is associated with each molecule of the compound, and it is the nitrogen atom that is part of a double bond in formula I that is protonated, i.e. associated with the tartaric acid. The precipitated tartrate salt may be solvated, i.e. the salt may contain some solvent. When the tartrate salt is precipitated from methanol, particularly when it is a tartrate salt of the compound of Example 2, it contains some methanol. Preferably, and particularly when the compound is the compound of Example 2 and its tartrate salt is precipitated from methanol, it is precipitated at a temperature of 25°-29°C, especially 27°C.

Also preferably, and particularly when the compound is the compound of Example 2 and its tartrate salt is precipitated from a methanolic solution having a temperature as set forth in the preceding paragraph, the solution has a maximum concentration of 0.3 M, preferably 0.21 M, of the two enantiomers together. In each case the minimum concentration is preferably 0.1 M, more preferably 0.19 M. The most preferred concentration is 0.20 M.

Resolution is preferably carried out by forming a solution of the D-(-)-tartrate or L-(+)-tartrate salts of such a racemic compound, selectively precipitating one enantiomer, and neutralizing the precipitated enantiomer. The solution of the D-(-)- or L-(+)-tartrate salts of the racemic compound is formed by, for example, reacting said racemic compound with 0.98-2, preferably 1.0, mole of above acid per mole of the racemic compound in, preferably, methanol, at a temperature of 30°-60°C, preferably 35°-45°C. The temperature of the methanolic solution is initially as set forth in this paragraph, and the temperature to which it is adjusted to effect the selective precipitation and its concentration are as set forth above. The precipitated tartrate salt is isolated and neutralized by treatment with, for example, at least one equivalent of a base, preferably an inorganic base such as sodium or potassium hydroxide, carbonate or bicarbonate. If desired, the obtained single enantiomer is then isolated.

Preferably, the D-(-)- or L-(+)-tartrate salts are monoacid addition salts of a 5R,5S racemate of a compound having just one center of asymmetry, preferably the compound of Example 2, the methanolic solution of said salts has a temperature of 30°-65°C and a maximum concentration of 0.3 M of the two enantiomers together,

and the temperature of the solution is adjusted to 25°-29°C to effect the selective precipitation. More preferably, the methanolic solution has a temperature of 35°-45°C and a concentration of 0.15-0.22 M, especially 0.20 M, prior to adjustment of the temperature to 26.5°-27.5°C, especially 27°C, to effect the selective precipitation.

If necessary and/or desired, the precipitated tartrate salt or the isolated free base may be recrystallized one or more times to obtain a purer product.

A mixture of tartrate salts enriched with the non-precipitated enantiomer may be obtained from the mother liquor and washings from which additional quantities of the desired (i.e. precipitated) enantiomer may be obtained, before, after or without isolating the undesired (i.e.non-precipitated) enantiomer. Preferably, however, the mother liquor and washings are combined and neutralized to obtain a mixture of the free bases enriched with the other (i.e. undesired or non-precipitated) enantiomer, the mixture of free bases is reacted with the other tartaric acid [i.e. L-(+)-tartaric acid if the mixture of free bases is enriched with the 5S enantiomer and D-(-)-tartaric acid if it is enriched with the 5R enantiomer] to form a mixture of tartrate salts, the undesired enantiomer is selectively precipitated, the resulting mother liquor and washings are combined and neutralized to obtain a mixture of free bases now enriched with the desired enantiomer and the original procedure is repeated to obtain an additional quantity of the desired enantiomer. The "recycling" may be repeated one or more times to obtain additional quantities of the desired enantiomer.

Alternatively, the undesired enantiomer of the tartrate salts is first precipitated, and a mixture of tartrate salts enriched with the desired enantiomer is obtained from the mother liquor and/or the combined mother liquor and washings. The mixture of tartrate salts enriched with the desired enantiomer is then neutralized to form a mixture of free bases enriched with the desired enantiomer which is used as the starting material in lieu of a racemate to obtain a larger quantity of the desired isomer than had the racemate itself been utilized with no "recycling".

The starting materials may be obtained in conventional manner.

The compounds of formula II may e.g. be prepared by reaction of 2-aminobenzophenone or an appropriately substituted 2-aminobenzophenone derivative with a reducing agent such as sodium borohydride and reaction of the resultant $\alpha$-(2-aminophenyl)benzenemethanol derivative with e.g. ammonium thiocyanate under acidic conditions.

The compounds of formula II may be prepared in optically active form e.g. by reacting 2-aminobenzophenone or an appropriately substituted 2-aminobenzophenone derivative with hydroxylamine hydrochloride, reacting the resultant 2-(hydroxyiminophenylmethyl)benzeneamine derivative with a reducing agent such as powdered zinc in ammonium acetate and ethanol in the presence of ammonium hydroxide, followed by salt formation of the resultant racemic $\alpha$-(2-aminophenyl)benzylamine derivative with either D-(-)- or L-(+)-tartaric acid and fractionation of the resultant corresponding diastereoisomeric $\alpha$-(2-aminophenyl)benzylammonium tartrate salts to give the optically pure tartaric salts, conversion to the optically pure $\alpha$-(2-aminophenyl)benzylamine with e.g. sodium carbonate or bicarbonate, and cyclization to the corresponding compound of formula II in optically active form with e.g. thiophosgene and an organic base or 1,1'-thiocarbonyldiimidazole in an inert organic solvent.

Insofar as its preparation is not specifically described herein, a compound used as a starting material is known or may be prepared by known methods starting from known compounds, e.g. as described in the Examples.

The following Examples illustrate the invention. Temperatures are in degrees Centigrade. m.p. = melting point; Me = methyl; Phe = phenyl; Et = ethyl; b = in free base form. Optical rotation: c is given in g/dl.

**Example 1: (R)- and (S)-3-Methyl-5-phenyl-5H-thiazolo[2,3-b]quinazoline**

[Formula I: A + B = -C($R_4R_5$)C($R_6R_7$)-S-, with the carbon atom carrying $R_4$ and $R_6$ bound to the nitrogen atom;
$R_1$, $R_2$, $R_3$, $R_7$, $R_6$, $R_9$ = H; $R_4$ = Me;
$R_6$ + $R_6$ = bond;
in R or, respectively, S configuration at C5;
in free base and hydrobromide salt form]

[Process variant a) (dehydration), and conversion of salt into free base]

a) Hydrobromide salt: A suspension of 42 g of **(5R)-2,3-dihydro-3-methyl-5-phenyl-5H-thiazolo[2,3-b]quinazolin-3-ol hydrobromide** (R-isomer of compound of Example 7) in 600 ml of 2-propanol is slowly heated to reflux and refluxed for 2.5 hours, and the volume is reduced at reduced pressure to obtain **(R)-3-methyl-5-phenyl-5H-thiazolo[2,3-b]quinazoline hydrobromide.**

The **S enantiomer in hydrobromide salt form** is obtained in analogous manner.

b) Free base: 200 ml of saturated sodium chloride solution is added to the **product obtained under a)**

above, 300 ml of 2N sodium carbonate solution is added, and the reaction mixture is stirred at 20-25° for 5 minutes and extracted three times with methylene chloride. The methylene chloride extracts are combined, dried over anhydrous sodium sulfate, filtered and evaporated at reduced pressure until the solution becomes cloudy. Anhydrous diethyl ether is added with cooling at 0°-5°, and the resulting solid is collected by filtration, washed with petroleum ether and vacuum dried at 40° for 5 hours to obtain the **R title compound** [m.p. 177°-179°; $[\alpha]_D^{25}$ = -181.1° (c = 1, $CH_3OH$)].

The **S title compound** is obtained in analogous manner [99.45 % S product; m.p. 173-176°; $[\alpha]_D^{25}$ = + 151.8° (c = 1, $CH_3OH$)].

**Example 2: (±)-3-Methyl-5-phenyl-5H-thiazolo[2,3-b]quinazoline**

[Formula I: as for Example 1;
in racemate form;
in free base and hydrochloride salt form]

[Process variant b) (ring closure)]

a) **Hydrochloride salt:** 13.7 g of sodium bromide is added to a suspension of 173.7 g of **(±)-3,4-dihydro-4-phenyl-2(1H)-quinazolinethione** (compound of formula II) in 965 ml of 2-propanol, the resulting suspension is heated to 75° with stirring, 75.2 ml of 96 % **chloroacetone** is added over a 10 minute period to the suspension stirred at 75°-78°, the addition being mildly exothermic, and the resulting white suspension is stirred at 75° for 3 hours, the reaction mixture being stirred under nitrogen throughout. As much of the solvent as possible is removed by distillation at 40-45° and 175-190 mmHg to obtain a thick stirrable slurry containing the **title compound in hydrochloride salt form,** which is used further without isolation of the product.

b) **Free base:** 565 ml of ethyl acetate is added to the thick stirrable **slurry obtained under a)** above, the resulting suspension is heated to 45° and, over a period of 10 minutes, a solution of 126 g of sodium bicarbonate in 1.13 l of water is added dropwise with stirring at 35°-45°. The resulting light beige suspension is stirred at 45° for 1 hour, cooled to 20° and vacuum filtered. The filter cake is washed with two 625 ml portions of warm (about 50°) water and then with two 110 ml portions of cold (about 0°) ethyl acetate, and the damp solid is suspended in 2.1 l of acetonitrile. The suspension is heated to gentle reflux (about 81°) to obtain a solution, the solution is cooled to 10° over a period of about 15 minutes, and the resulting suspension is stirred at 10° for 1 hour and vacuum filtered. The filter cake is washed with 250 ml of cold (about 0°) acetonitrile, and the solid is collected and vacuum dried at 45-50° to constant weight to obtain the pale, light amber solid **title compound in free base form** (m.p. 184°-185°).

The starting material is obtained as follows:

a) 30.4 g of sodium borohydride pellets is added portionwise over a 5 minute period to a mixture of 301 g of 98 % **2-aminobenzophenone** and 650 ml of 95 % ethanol stirred at 65°, the addition being mildly exothermic, and the reaction mixture is stirred at 65°-70° for 1.5 hours to obtain an off-white suspension, the reaction mixture being stirred under nitrogen throughout. 550 ml of water is added over a period of 10 minutes with stirring at 60°-65°, the addition being slightly endothermic, to obtain a white mixture containing **(±)-α-(2-aminophenyl)benzenemethanol** which is used further without isolation.

b) A solution of 125 g of ammonium thiocyanate in 250 ml of water is added over a period of 20 minutes to the white **mixture obtained under a) above,** stirred at 65°-68°, the addition initially being slightly exothermic. A solution of 205 ml of 36 % hydrochloric acid in 400 ml of water is added dropwise over a period of about 20 minutes with stirring at 65-70°, the addition being exothermic. The resulting bright white mixture is stirred at 65°-70° for 2 hours, cooled to 45° and vacuum filtered through a Büchner funnel having a polypropylene filter pad. The filter cake is washed with three 500 ml portions of warm (50°) water and then with two 300 ml portions of 95 % ethanol. The resulting damp solid is suspended in a mixture of 270 ml of methanol and 300 ml of water, 800 ml of water is added, and the suspension is stirred at 50° for 45 minutes and filtered at this temperature through a Büchner funnel having a polypropylene filter pad. The resulting filter cake is washed with two 250 ml portions of warm (about 50°) water and then with 150 ml of cold (about 5°) methanol. The solid is collected and vacuum dried at 40°-45° to constant weight (about 16 hours) to obtain the 100 % pure **(±)-3,4-dihydro-4-phenyl-2(1H)-quinazolinethione** as a white solid (m.p. 226°-229°).

**Example 3: (R)- and (S)-3-methyl-5-phenyl-5H-thiazolo[2,3-b]quinazoline [S-(R*, R*)]-2,3-dihydroxybutanedioic acid salt**

[Formula I: as for Example 1;

in R or, respectively, S configuration at C5;
in D-(-)-tartaric acid salt form]

[Conversion into optically active salt]

A suspension of 185 g of **title compound of Example 2** in 3.33 l of methanol is heated to 35°-40°, 100 g of **D-(-)-tartaric acid** is added portionwise over a 5 minute period during which the temperature is maintained at about 40°. The resulting solution is cooled to 27° and stirred at 27°-28° for 2 hours, the reaction mixture being stirred under nitrogen throughout. The suspension is filtered through a Büchner funnel having a polypropylene filter pad, the filter cake is washed with 200 ml of cold (about 0°) methanol, and the solid is collected and vacuum dried at 40°-45° to constant weight (about 16 hours) to obtain the 99.86 % pure **R-enantiomer** as a white solid [m.p. 99° (dec.); $[\alpha]_D^{25}$ = -108.51° (c = 1, $CH_3OH$)].

An approximately 3:7 mixture of the **R and the S enantiomer** may be obtained from the combined mother liquors and washings.

**Example 4: (R)-3-Methyl-5-phenyl-5H-thiazolo[2,3-b]quinazoline**

[Formula I; as for Example 1;
in R configuration at C5;
in free base form]

[Conversion of salt into free base]

A solution of 56.3 g of sodium bicarbonate in 570 ml of water is added dropwise over a 15 minute period to a suspension of 75.0 g of **title compound of Example 3, R-isomer** in 875 ml of ethyl acetate stirred at 35°, the resulting biphasic mixture is stirred at 35°-40° for 1 hour, and the (upper) organic phase is separated, washed three times with 100 ml portions of warm (about 30°-35°) water and concentrated by vacuum distillation at 45° and 100 mmHg to obtain a fluid residue. 375 ml of n-heptane is added, and the mixture is cooled to -10°, stirred at this temperature for 15 minutes and vacuum filtered. The filter cake is washed with 100 ml of cold (about 0°) n-heptane, and the solid is collected and vacuum dried at 50°-55° to constant weight (about 16 hours) to obtain the 99.93 % pure **title compound** as a white solid [m.p. 177°-179°; $[\alpha]_D^{25}$ = -181.1° (c = 1, $CH_3OH$)].

**Example 5: (R)-3-Methyl-5-phenyl-5H-thiazolo[2,3-b]quinazoline hydrochloride**

[Formula I: as for Example 1;
in R configuration at C5;
in hydrochloride salt form]

[Conversion of free base into salt]

A solution of 1.42 g of anhydrous hydrogen chloride in 33.3 ml of anhydrous diethyl ether is added dropwise over a period of 10 minutes to a solution of 11.0 g of **title compound of Example 4** in 300 ml of dry tetrahydrofuran stirred at 5°, 300 ml of anhydrous diethyl ether is added, the reaction mixture is stirred for 10 minutes, and the precipitate is collected by filtration and vacuum dried to obtain the **title compound** [m.p. 238°-241°].

**Example 6: (±)-7-Chloro-3-methyl-5-phenyl-5H-thiazolo[2,3-b]quinazoline**

[Formula I: A + B = -C($R_4R_5$)C($R_6R_7$)-S-, with the carbon atom carrying $R_4$ and $R_6$ bound to the nitrogen atom;
$R_1$, $R_2$, $R_3$, $R_7$, $R_9$ = H; $R_4$ = Me;
$R_6$ + $R_6$ = bond;
$R_6$ = 7-Cl (i.e. is separated by 4 carbon atoms from each of the two nitrogen atoms in formula I);
in racemate form;
in free base and hydrobromide salt form]

[Process variant a) (dehydration) and conversion of salt into free base]

**a) Hydrobromide salt:** The **title compound in hydrobromide salt form** is prepared analogously to Example 1a) starting from the **compound of Example 8.**

**b) Free base:** The **title compound in free base form** (m.p. 220°-223°) is obtained analogously to Example 1b), starting from the **product of step a)** above.

The title compound in free base and hydrobromide salt form is a racemate that may be resolved to obtain the **5R and 5S enantiomers.** Alternatively, the two enantiomers may be prepared analogously to Examples 7 and B starting from single isomers.

**Example 7: (5R)- and (5S)-2,3-Dihydro-3-methyl-5-phenyl-5H-thiazolo-[2,3-b]quinazolin-3-ol hydrobromide**

[Formula I: A + B = -C($R_4R_5$)C($R_6R_7$)-S-, with the carbon atom carrying $R_4$ and $R_6$ bound to the nitrogen atom;
$R_1$, $R_2$, $R_3$, $R_6$, $R_7$, $R_8$, $R_9$ = H; $R_4$ = Me;
$R_6$ = OH;
in R or, respectively, S configuration at C5;
in hydrobromide salt form]

[Process variant b) (ring closure)]

26.7 g of **bromoacetone** dried over anhydrous sodium sulfate is added dropwise over a period of 5 minutes to a solution of 29.6 g of **(R)-3,4-dihydro-4-phenyl-2(1H)-quinazolinethione** (compound of formula II) in a mixture of 400 ml of dry tetrahydrofuran and 170 ml of anhydrous diethyl ether stirred at -20 to -10°, and the reaction mixture is allowed to warm 20°-25° and stirred at this temperature for 64 hours, the reaction mixture being maintained under nitrogen throughout. The resulting solid is collected by filtration, washed with dry tetrahydrofuran, washed with anhydrous diethyl ether and vacuum dried at 40° for 5 hours to obtain the **5R title compound** [m.p. 162°-163° (dec.); a previous batch melted at 168°-170° (dec.)].

This compound may be a mixture of two diastereoisomers that may be separated by conventional means to obtain the **3R,5R** and **3S,5R enantiomers.**

The **5S title compound** [m.p. 159°-160° (dec.)] is obtained in analogous manner from the corresponding S starting material. It may be a mixture of two diastereoisomers that may be separated by conventional means to obtain the **3R,5S** and **3S,5S enantiomers.**

The **R starting material** (for the 5R title compound) is obtained as follows:

a) 478.85 g of **hydroxylamine hydrochloride** is added to 400 g of **2-aminobenzophenone** in 3 l of absolute ethanol, 470 ml of pyridine is added, and the reaction mixture is gently refluxed for 16 hours, allowed to cool and evaporated at reduced pressure to about 25 % of its original volume. The resulting thick dark green oil is added to ethyl acetate, and working up effected in conventional manner obtain **2-(hydroxyiminophenylmethyl)benzeneamine** (m.p. 154°-157°; another batch melted at 157°-159°).

b) 77 g of ammonium acetate is added to 285.63 g of **product of step a)** above in 1.2 l of 95 % ethanol, 538 g of powdered **zinc** is added, 2.25 l of 29 % ammonium hydroxide solution is added, and the reaction mixture is warmed to 38°, maintained at this temperature for 1.5 hours, gently refluxed for 16 hours, cooled to 60° and filtered. The zinc is washed with ethyl acetate, and the ethyl acetate washing is added to the filtrate. 500 ml of 2N sodium hydroxide solution and 1 l of saturated sodium chloride solution are added to the filtrate. The mixture is worked up in conventional manner obtain crude **(±)-α-(2-aminophenyl)benzylamine** as an oil.

c) 165.1 g of **D-(-)-tartaric acid** is added to a solution of 217.8 g of **product of step b)** above in 3.4 l of 98.5 % ethanol stirred at 50°. The reaction mixture is refluxed for 30 minutes, 500 ml of 95 % ethanol is added, and the mixture is refluxed for 3 hours and filtered hot (70°-78°). The solid is washed with 400 ml of hot (70°-78°) absolute ethanol, washed with petroleum ether and vacuum dried at 40° for 5 hours to obtain the crude product. The crude product is added to 1.7 l of absolute ethanol, the mixture is refluxed for 1 hour and filtered hot (70°-78°), and the solid is washed with 250 ml of hot (70-78°) absolute ethanol, washed with 200 ml of petroleum ether and vacuum dried at 40° for 5 hours to obtain **(R)-α-(2-ammoniumphenyl)benzylammonium D-(-)-tartrate** (94.58 % R product).

d) 500 ml of ethyl acetate is added to 80.27 g of **product of step c)** above in 500 ml of water and 500 ml of saturated sodium chloride solution, 600 ml of 10 % sodium bicarbonate solution is added, the reaction mixture is stirred at 20°-25° for 10 minutes, the ethyl acetate layer is separated, and the aqueous layer is reextracted twice with ethyl acetate. The three ethyl acetate layers are combined, dried over anhydrous sodium sulfate, filtered and evaporated to dryness at reduced pressure to obtain **(R)-α-(2-aminophenyl)benzylamine** (94.58 % R product).

e) 33.5 g of **1,1'-thiocarbonyldiimidazole** is added over a period of 20 minutes to a solution of 35.5 g of **product of step d)** above in 500 ml of HPLC grade methylene chloride stirred at -40° to -30°, and the reaction mixture is allowed to warm to 20°-25° with stirring and stirred at 20-25° for 16 hours, the reaction mixture being maintained under nitrogen throughout. The reaction mixture is extracted with a 1:1 mixture of saturated sodium chloride solution and water, and the aqueous layer is reectracted three times with methylene chloride. The four methylene chloride layers are combined, dried over anhydrous sodium sulfate, filtered and evaporated at reduced pressure until the solution becomes cloudy. The cloudy solution is cooled to 0°-5°, and the resulting solid is collected by filtration and vacuum dried at 40° for 5 hours to obtain **(R)-3,4-dihydro-4-phenyl-2(1H)-quinazolinethione** (more than 97.3 % R product; m.p. 207-211°). The

filtrate is partially evaporated at reduced pressure and cooled, and the resulting solid is collected by filtration and vacuum dried at 40° for 5 hours to obtain a second crop.

The corresponding **S starting material** (for the 5S title compound) is obtained analogously (m.p. 205°-209°) using for step c) the following conditions:

c') 202.6 g (1.35 moles) of **L-(+)-tartaric acid** is added to a solution of 269.4 g (1.35 moles) of **(±)-α-(2-aminophenyl)benzylamine** in 5 l of about 97.6 % ethanol stirred at 50°, and the reaction mixture is heated to reflux over a period of about 30 minutes (a precipitate forming at about 60°), refluxed for 30 minutes, cooled to 75° and maintained at this temperature for 1 hour. 800 ml of absolute ethanol is added, and the solid is collected by filtration (while warm), washed with 600 ml of hot (70°-78°) absolute ethanol, washed with 200 ml of petroleum ether and dried at 40° under high vacuum for 5 hours to obtain crude **(S)-α-(2-ammoniumphenyl)benzylammonium L-tartrate** (85.67 % S). The crude product is added to 2 l of absolute ethanol, the mixture is refluxed for 1 hour and filtered while hot (70°-78°), and the collected solid is washed with 300 ml of hot (70°-78°) absolute ethanol, washed with petroleum ether and vacuum dried at 40° for 5 hours to obtain 91.39 % S crude product. The crude product is added with rapid agitation to 4.5 l of absolute ethanol, the mixture is refluxed for 2 hours and filtered while hot (70°-78°), and the collected solid is washed with 400 ml of hot (70-78°) 95 % ethanol, washed with petroleum ether and vacuum dried at 40° for 5 hours. The filtrates and washings are combined, evaporated at reduced pressure to a total volume of 4.25 l and allowed to stand for 16 hours at 20°-25°. The resulting solid is collected by filtration, washed with petroleum ether and vacuum dried at 40° for 5 hours to obtain 98.8 % **S product** (29.31 g). The filtrates and washings are combined and evaporated at reduced pressure to about 50 % of the original volume and allowed to stand for 16 hours at 20°-25°. The resulting solid is collected by filtration, washed with petroleum ether and vacuum dried at 40° for 5 hours to obtain a second crop (95.5 % S product).

**Example 8: (±)-7-Chloro-2,3-dihydro-3-methyl-5-phenyl-5H-thiazolo[2,3-b)quinazolin-3-ol hydrobromide**

[Formula I: A + B = -C($R_4R_5$)C($R_6R_7$)-S-, with the carbon atom carrying $R_4$ and $R_6$ bound to the nitrogen atom;
$R_1$, $R_2$, $R_3$, $R_6$, $R_7$, $R_9$ = H; $R_4$ = Me;
$R_6$ = OH;
$R_6$ = 7-Cl (i.e. is separated by 4 carbon atoms from each of the two nitrogen atoms in formula I);
in racemate form;
in hydrobromide salt form)]

[Process variant b) (ring closure)]

The **title compound** [m.p. 312°-314° (dec.)] is obtained analogously to Example 7, starting from **(±)-7-chloro-3,4-dihydro-4-phenyl-2(1H)-quinazolinethione** (compound of formula II). It is a mixture of stereoisomers which may be separated by conventional means.

The starting material is obtained as follows:

a) 40.85 g of sodium borohydride is added portionwise to a solution of 250 g of **2-amino-5-chlorobenzophenone** in 2.5 l of HPLC grade methanol stirred at 0° to 5° over a period of 4 hours, and the reaction mixture is allowed to warm to 20°-25° and stirred at this temperature for 16 hours, the reaction mixture being maintained under nitrogen throughout. Ice and sufficient acetic acid to adjust the pH to 6 are added, and working up effected in conventional manner **(±)-α-(2-Amino-5-chlorophenyl)benzenemethanol** is obtained (m.p. 104°-107°).

b) 85 ml of concentrated hydrochloric acid is added dropwise to 199.01 g of **compound of step a) above** in 1.2 l of water over a period of 30 minutes at 20°-25°, 70.3 g of ammonium thiocyanate is added in small portions, and the reaction mixture is slowly heated to reflux with rapid agitation, refluxed for 2.5 hours, allowed to cool to 20°-25° and rapidly stirred at this temperature for 16 hours. The resulting solid is collected by filtration, washed with water, air dried and added to 3.5 l of methanol. The mixture is refluxed for 30 minutes and allowed to cool to 20°-25°, and the solid is collected by filtration, washed with petroleum ether and vacuum dried at 40° for 5 hours to obtain **(±)-6-chloro-3,4-dihydro-4-phenyl)-2(1H)-quinazolinethione** (m.p. 221-223°). The filtrate is evaporated at reduced pressure to about 50 % of its original volume, and the resulting solid is collected by filtration, washed with petroleum ether and vacuum dried at 40° for 5 hours to obtain a second crop (m.p. 226-228°).

**Example 9: Methyl (±)-(6-chloro-3,4-dihydro-4-phenylquinazolin-2-yl-thio)acetate hydrobromide**

[Formula I: A, $R_1$, $R_2$, $R_3$, $R_9$ = H;
B = -S-$CHR_7$'-$R_{10}$ ($R_7$' = H; $R_{10}$ = -COOMe);
$R_8$ = 6-Cl (i.e. is separated by 4 carbon atoms from each of the two nitrogen atoms in formula I);
in racemate form;
in hydrobromide salt form]
[Process variant c) (alkylation)]

16.8 g of **methyl bromoacetate** is added to a solution of 15.0 g of **(±)-6-chloro-3,4-dihydro-4-phenyl-2(1H)-quinazolinethione** [see Example 8, step b)] in 150 ml of dry tetrahydrofuran stirred at 20°-25°, and the reaction mixture is stirred at 20°-25° for 48 hours, the reaction mixture being maintained under nitrogen throughout. The resulting solid is collected by filtration, washed with dry tetrahydrofuran, washed with petroleum ether and vacuum dried at 40° for 5 hours to obtain the **title compound** [m.p. 215°-219° (dec.)]

The product is a racemate that may be resolved to obtain the **4R and the 4S enantiomers.** Alternatively, the two enantiomers may be prepared from single isomers of the starting material.

The following compounds of the invention may be prepared in analogous manner:

a) Compounds of formula I wherein A and B together are a group $-C(R_4R_5)C(R_6R_7)-S-$:

| Example | $R_1$ | $R_2$ | $R_3$ | $R_4$ | $R_5$ | $R_6$ | $R_7$ | $R_8$ | $R_9$ | Isomers | Salt | m.p. |
|---|---|---|---|---|---|---|---|---|---|---|---|---|
| 10 | H | H | H | Phe | OH | H | H | 7-Cl | H | mixture[1] | HBr | 165°-169° (dec.) |
| 11 | H | H | H | Et | OH | H | H | 7-Cl | H | mixture[1] | HBr | 161°-162° (dec.) |
| 12 | H | H | H | Me | OH | H | H | 7-Me | H | mixture[1] | HBr | 287°-289° (dec.) |
| 13 | H | H | H | Phe | OH | H | H | H | H | mixture[1] | HBr | 77°- 81° (dec.) |
| 14 | 2-Cl | H | H | Me | OH | H | H | 7-Cl | H | mixture[1] | HBr | 290° (dec.) |
| 15 | 4-Cl | H | H | Me | OH | H | H | H | H | mixture[1] | HBr | 95°-130° (dec.) |
| 16 | H | H | H | $-CH_2Br$ | bond | | H | 7-Cl | H | racemate[2] | b | 265°-270° (dec.) |
| 17 | H | H | H | Me | bond | | H | 7-Cl | H | 5S[4] | b | 197°-200° a) |
| 18 | H | H | H | Et | bond | | H | 7-Cl | H | racemate[2] | b | 170°-172° (dec.) |
| 19 | H | H | H | Me | bond | | H | 7-Cl | H | 5R[4] | b | 195°-197° b) |
| 20 | H | H | H | Me | bond | | H | 7-Me | H | racemate[2] | b | 234°-238° |
| 21 | H | H | H | H | bond | | H | 7-Cl | H | racemate[2] | b | 174°-178° (dec.) |
| 22 | H | H | H | Me | bond | | H | 8-Me* | H | racemate[2] | b | 148°-155° |
| 23 | H | H | H | Phe | bond | | H | H | H | racemate[2] | b | 161°-164° |
| 24 | H | H | H | Me | bond | | H | H | H | 5R[4] | citrate | 75°- 80° (dec.) |
| 25 | H | H | H | Et | bond | | H | H | H | racemate[2] | b | 187°-189° |
| 26 | 2-Cl | H | H | Me | bond | | H | 7-Cl | H | racemate[2] | b | 215°-225° (dec.) |
| 27 | 4-Cl | H | H | Me | bond | | H | H | H | racemate[2] | b | 178°-180° |
| 28 | 2-F | H | H | Me | bond | | H | 7-Br | H | racemate[2] | b | 179°-181° |

b) Compounds of formula I wherein A is hydrogen and B is a group $-S-CHR_7'-R_{10}$:

| Example | $R_1$ | $R_2$ | $R_3$ | $R_7'$ | $R_{10}$ | $R_8$ | $R_9$ | Isomers | Salt | m.p. |
|---|---|---|---|---|---|---|---|---|---|---|
| 29 | H | H | H | H | vinyl | 6-Cl | H | racemate[3)] | HBr | 200°-208° |
| 30 | H | H | H | H | Et | 6-Cl | H | racemate[3)] | HBr | 205°-208° (dec.) |
| 31 | H | H | H | H | -COOMe | 6-Cl | H | 4S[4)] | HBr | 137°-140° (dec.)[c)] |
| 32 | H | H | H | H | -COOMe | 6-Cl | H | 4R[4)] | HBr | 135°-137° (dec.)[d)] |
| 33 | H | H | H | H | -COOMe | H | H | racemate[3)] | HBr | 194°-196° (dec.) |

* i.e. the methyl group is separated by 5 carbon atoms from the isolated phenyl ring in formula I

a) $[\alpha]_D^{25}$ = +25.5° (c=1, $CH_3OH$)
b) $[\alpha]_D^{25}$ = -24.5° (c=1, $CH_3OH$)
c) $[\alpha]_D^{25}$ = -128.58° (c=1, $CH_3OH$)
d) $[\alpha]_D^{25}$ = +134.06° (c=1, $CH_3OH$)

1) Mixture of isomers that may be separated;
2) 5R,5S racemate which may be resolved to obtain the individual enantiomers; alternatively, the individual enantiomers may be synthesized by the process variants set forth above;
3) 4R,4S racemate which may be resolved to obtain the individual enatiomers; alternatively, the individual enantiomers may be synthesized by the process variants set forth above;
4) May contain a small amount of its antipode.

The compounds of formula I in racemic or optically active, in free base or pharmaceutically acceptable acid addition salt form, hereinafter briefly named "the agents of the invention", possess interesting pharmacological activity. They are indicated for use as pharmaceuticals.

In particular, they raise the blood serum high density lipoprotein (HDL; HDL-cholesterol; and apolipoprotein A-I (Apo A-I)] level. Many of the compounds have the added benefit of lowering the blood serum total triglycerides level.

This activity can be determined in conventional assay methods, e.g. as follows:

a) Test A: In vivo HDL-cholesterol test:

The test is effected according to the method described in the literature, e.g. in EP 528 146

Other conventional methods may be used to assay the blood serum samples for HDL-cholesterol and total triglycerides content.

b) Test B: Apo A-I test:

The test is effected as described in the literature, e.g. in EP 528 146.

The above tests A and B indicate that the agents of the invention are active at a dosage in the range of from about 10 mg/kg to about 200 mg/kg per day.

The agents of the invention are therefore indicated for use in raising the blood serum high density lipoprotein (HDL; HDL-cholesterol and apolipoprotein A-I) level and many of them also for lowering the blood serum total triglycerides level and thus in the treatment of atherosclerosis.

The precise dosage to be employed depends of course upon several factors including the host, the nature and the severity of the condition being treated, the mode of administration and the particular agent employed. However, in general, satisfactory results are indicated to be obtained at daily dosages in the range of from about 400 mg to about 2000 mg, conveniently administered in divided doses two to four times a day.

The compounds may be administered by any conventional route, in particular enterally, preferably orally e.g. in the form of tablets or capsules, or parenterally e.g. in the form of sterile injectable solutions or suspensions.

The agent of the invention of Examples 1, 2, 3, 5 and 24, particularly in the R optically active form, is preferred.

Thus e.g. the following activity has been determined:

| Compound | Average dose (mg/kg/day) | Duration of treatment (days) | HDL-cholesterol level increase (%) | Apo-I level increase (%) | Total triglyceride level decrease (%) |
|---|---|---|---|---|---|
| Example 1, R-isomer (free base) | 83 | 8 | 402 | 62 | 15 |
| Gemfibrozil | 53 | 8 | 211 | 88 | 15 |

It is therefore indicated that, for this use, the agent of Example 1, particularly the R isomer may be administered at dosages lower than those conventionally employed with gemfibrozil by similar modes of administration, i.e. about 400-1000 mg/day orally.

Pharmaceutical compositions comprising an agent of the invention together with at least one pharmaceutically acceptable carrier or diluent, may be manufactured in conventional manner. They may be e.g. in unit dosage form containing, for example, from about 100 mg to about 500 mg of active compound.

The invention thus concerns a method for the prophylactic or curative treatment of atherosclerosis comprising administering to a patient in need of such treatment a therapeutically effective amount of an agent of the invention.

It further concerns a process for the preparation of a medicament against atherosclerosis which comprises mixing an agent of the invention with at least one pharmaceutically acceptable carrier or diluent.

It further comprises the use of an agent of the invention for the manufacture of a medicament against atherosclerosis.

The invention also concerns a pharmaceutical composition comprising an agent of the invention together with at least one pharmaceutically acceptable carrier or diluent.

It further comprises an agent of the invention for use as a pharmaceutical, particularly for use in the prophylactic or curative treatment of atherosclerosis.

## Claims

1. A compound of formula I

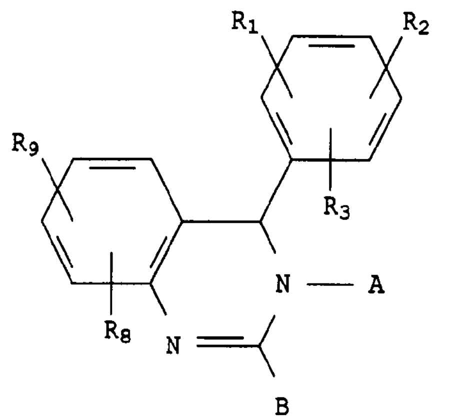

I

wherein

$R_1$ is hydrogen; alkyl of 1 to 3 carbon atoms; alkoxy of 1 to 3 carbon atoms; trifluoromethyl; or fluoro or chloro;

$R_2$ and $R_9$ independently are hydrogen; alkyl of 1 to 3 carbon atoms; alkoxy of 1 or 2 carbon atoms; trifluoromethyl; or fluoro or chloro;

$R_3$ is hydrogen or alkyl of 1 or 2 carbon atoms;

either A and B together are a group of formula $-C(R_4R_5)C(R_6R_7)-S-$ wherein

$R_4$ is hydrogen; alkyl of 1 to 3 carbon atoms optionally mono- or independently di- or independently trisubstituted by halogen of atomic number of from 9 to 35, whereby there is at most 1 bromo; or phenyl optionally mono- or independently disubstituted by alkyl of 1 to 3 carbon atoms, alkoxy of 1 or 2 carbon atoms, or halogen of atomic number of from 9 to 35, whereby there is at most 1 bromo;

$R_5$ is hydroxy and $R_6$ is hydrogen or

$R_5$ and $R_6$ together form a double ($\pi$) bond; and

$R_7$ is hydrogen or alkyl of 1 or 2 carbon atoms;

or A is hydrogen and B is a group of formula $-S-CHR_7'-R_{10}$ wherein

$R_7'$ is hydrogen or alkyl of 1 or 2 carbon atoms and

$R_{10}$ is alkyl of 1 to 3 carbon atoms; alkenyl of 2 or 3 carbon atoms; or $-COOR_{11}$ wherein $R_{11}$ is the residue of an ester group, with the proviso that $R_{10}$ is other than methyl when $R_7'$ is hydrogen;

$R_8$ is hydrogen; alkyl of 1 to 3 carbon atoms; alkoxy of 1 to 3 carbon atoms; trifluoromethyl; or halogen of atomic number of from 9 to 35;

in racemic or optically active, in free base or acid addition salt form.

2. A compound according to claim 1 of formula Is

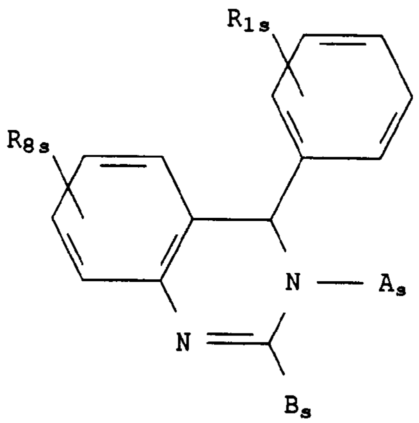

Is

wherein

$R_{1s}$ is hydrogen, fluoro or chloro;

either $A_s$ and $B_s$ together are a group of formula $-C(R_{4s}R_5)CH(R_6)-S-$ wherein

$R_{4s}$ is hydrogen; alkyl of 1 or 2 carbon atoms optionally monosubstituted by bromo; or phenyl; and

$R_5$ and $R_6$ are as defined above;

or $A_s$ is hydrogen and $B_s$ is a group of formula $-S-CH_2-R_{10s}$ wherein

$R_{10s}$ is alkyl of 2 or 3 carbon atoms, alkenyl of 2 or 3 carbon atoms or $-COOR_{11s}$ wherein $R_{11s}$ is alkyl of 1 or 2 carbon atoms;

$R_{8s}$ is hydrogen; alkyl of 1 or 2 carbon atoms; or halogen of atomic number of from 9 to 35;

in racemic or optically active, in free base or acid addition salt form.

3. A compound according to claim 1 wherein A and B together are a group of formula $-C(R_4R_6)C(R_6R_7)-S-$, wherein $R_5$ is hydroxy, $R_2$, $R_3$, $R_6$, $R_7$ and $R_9$ are hydrogen, and $R_1$, $R_4$ and $R_8$ respectively are

- either hydrogen, methyl and hydrogen and the compound is in 5R or 5S optically active form;
- or hydrogen, methyl and 7-chloro,
  hydrogen, phenyl and 7-chloro,
  hydrogen, ethyl and 7-chloro,
  hydrogen, methyl and 7-methyl,
  hydrogen, phenyl and hydrogen,
  2-chloro, methyl and 7-chloro or
  4-chloro, methyl and hydrogen;
  and the compound is in racemic form;

or wherein $R_6$ and $R_6$ together form a double ($\pi$) bond, $R_2$, $R_3$, $R_7$ and $R_9$ are hydrogen, and $R_1$, $R_4$ and $R_8$ respectively are

- either hydrogen, methyl and 7-chloro or
  hydrogen, $-CH_2Br$ and 7-chloro;
  and the compound is in racemic form;
- or hydrogen, methyl and 7-chloro
  and the compound is in 5S or 5R optically active form;
- or hydrogen, ethyl and 7-chloro,
  hydrogen, methyl and 7-methyl,
  hydrogen, hydrogen and 7-Cl,
  hydrogen, methyl and 8-methyl,
  hydrogen, phenyl and hydrogen,
  hydrogen, ethyl and hydrogen,
  2-chloro, methyl and 7-chloro,
  4-chloro, methyl and hydrogen or
  2-fluoro, methyl and 7-bromo;
  and the compound is in racemic form;

or a compound according to claim 1 wherein A is hydrogen and B is a group of formula $-S-CHR_7'-R_{10}$, wherein $R_1$, $R_2$, $R_3$, $R_7'$ and $R_9$ are hydrogen, and $R_6$ and $R_{10}$ respectively are,

- either 6-chloro and methoxycarbonyl and the compound is in racemic form;

- or 6-chloro and vinyl or ethyl and the compound is in racemic form;
- or 6-chloro and methoxycarbonyl and the compound is in 4S or 4R optically active form;
- or hydrogen and methoxycarbonyl and the compound is in racemic form;

in free base or acid addition salt form.

**4.** The compound 3-methyl-5-phenyl-5H-thiazolo[2,3-b]quinazoline in racemic or optically active, in free base or acid addition salt form.

**5.** The compound according to claim 4 in 5R optically active form, in free base or acid addition salt form.

**6.** The compound according to claim 5, in free base form.

**7.** A process for the preparation of a compound according to claim 1 which comprises
a) for the preparation of a compound of formula I wherein
$R_5$ and $R_6$ together form a double ($\pi$) bond,
dehydrating a corresponding compound of formula I wherein
$R_5$ is hydroxy and $R_6$ is hydrogen;
b) for the preparation of a compound of formula I as defined in claim 1, submitting to appropriate ring closure a compound of formula II

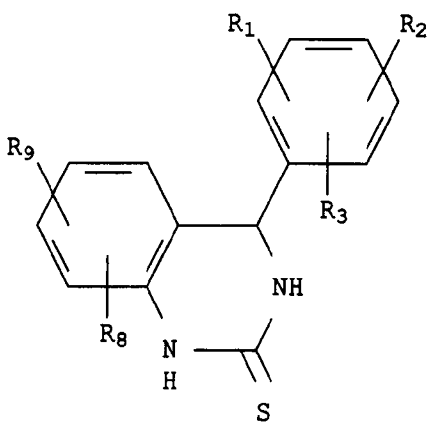

II

wherein the substituents are as defined in claim 1; or
c) for the preparation of a compound of formula I wherein
A is hydrogen and B is a group of formula $-S-CHR_7'-R_{10}$,
appropriately alkylating a corresponding compound of formula II,
e.g. with a corresponding compound of formula III

$$X'-CHR_7'-R_{10} \qquad III$$

wherein
X' is a reactive group and $R_7'$ and $R_{10}$ are as defined in claim 1;
and recovering the resultant compound of formula I in racemic or optically active, in free base or acid addition salt form.

**8.** A compound according to any one of claims 1 to 6 in racemic or optically active, in free base or pharmaceutically acceptable acid addition salt form, for use as a pharmaceutical.

**9.** A pharmaceutical composition comprising a compound of formula I as defined in claim 1 in racemic or optically active, in free base or pharmaceutically acceptable acid addition salt form, together with at least one pharmaceutically acceptable carrier or diluent.

**10.** Use of a compound of formula I as defined in claim 1 in racemic or optically active, in free base or pharmaceutically acceptable acid addition salt form for the manufacture of a medicament against atherosclerosis.

European Patent Office

# EUROPEAN SEARCH REPORT

Application Number

EP 93 81 0142

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | WO-A-8 903 215 (BOEHRINGER INGELHEIM)<br>* claims 1,2 * | 1,8,10 | C07D513/04<br>C07D239/78<br>A61K31/505<br>//(C07D513/04, 277:00,239:00) |
| A | CHEMICAL ABSTRACTS, vol. 89, no. 13, 25 September 1978, Columbus, Ohio, US; abstract no. 109566c, FUMIYOSHI ISHIKAWA ET AL 'Quinazolines' page 931 ;<br>* abstract *<br>& JP-A-53 044 592 (DAIICHI SEIYAKU) | 1,8 | |
| A | PHARMAZIE.<br>vol. 45, no. 10, October 1990, BERLIN DD pages 721 - 724<br>P. RICHTER ET AL 'Synthese von 2-Hydrazino -1,4-bzw.-3,4-Dihydrochinazolinderivaten'<br>* page 722, left column * | 1 | |
| A | GB-A-1 242 863 (PHARMACIA AS)<br>* claims 1,4 * | 1,8 | |

TECHNICAL FIELDS SEARCHED (Int. Cl.5 )

C07D
A61K

The present search report has been drawn up for all claims

| Place of search | Date of completion of the search | Examiner |
|---|---|---|
| THE HAGUE | 19 JULY 1993 | VOYIAZOGLOU D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)